# EUROPEAN PATENT APPLICATION

(11) **EP 1 747 802 A1**
(43) Date of publication of application: **31.01.2007**
(21) Application number: 05016047.2
(22) Date of filing: 23.07.2005
(51) Int. Cl.: A61Q 5/06, A61K 8/06, A61K 8/81, A61K 8/92

(54) **Hair styling composition in the form of a microemulsion**

(71) Applicant: Wella Aktiengesellschaft, 64274 Darmstadt (DE)
(72) Inventor: Stein, Bernd, 63768 Hösbach (DE); Baecker, Sabine, 65428 Rüsselsheim (DE); Runge, Martina, 68163 Mannheim (DE)

(57) **Abstract**

The compositions of the present invention relate to improved hair styling compositions in the form of microemulsions which are non-fluid and clear or transparent and which are comprising 20 to 60% by weight water; at least 3% by weight of an oil phase comprising liquid hydrophobic oils, at least 20% by weight of emulsifier; anionic hair fixing polymers and/or nonionic hair fixing polymers. Preferred polymers are anionic vinylacetate / crotonates copolymer and nonionic vinylpyrrolidone homopolymer.

## Description

### FIELD OF THE INVENTION

The present invention relates to a hair styling composition in the form of a microemulsion, which is non-fluid and clear or transparent and which comprises water, an oil phase, at least 20% by weight of emulsifier, anionic hair fixing polymer and/or nonionic hair fixing polymer.

### BACKGROUND OF THE INVENTION

Hair styling products are intended for helping to create individual hair styles and for temporarily holding them in place for a period of time. Gelwaxes play an important role among styling products providing shine and texture. Specific characteristics are their transparency and their non-fluid, highly viscous or semi-solid or solid consistency. They are often based on microemulsion with relativly high amount of emulsifier due to the high amount of emulsifier there is often an increased risk of skin or eye irritance. Reducing the amount of emulsifier impairs product clarity. It is a special challenge to formulate a clear product which is also sufficiently compatible for the eye mucous membrane. It is a further special challenge to provide a clear transparent gelwax with high product stability that gives hair good texture and increased hold. Hair hold and product stability can be achieved by high viscosity. However, this might have a negative impact on the convenience to work the product into the hair. Another option is to improve the hair hold factor by formulating with film forming or hair fixing polymers. But this again can impair product clarity because it is hard to get a clear product when incorporating a high molecular weight polymer into the microemulsion. Typical high molecular weight polymers with good hair fixing power are often not dissolved or dispersed in a microemulsion in a clear form or they impair the microemuslsion system, changing it to a non-transparent, milky emulsion. Furthermore, the high amounts of emulsifiers and oils which are necessary for the transparency of the microemulsion often reduce the hair fixing effect of hair styling polymers because they can act as plasticiers.

Microemulsion or gelwax products currently marketed have good properties either in giving hair gloss or giving hair texture or giving hair hold or in product transparency, rigidity and stability over a long time at elevated temperatures or in being of very low skin or mucous membrane irritancy or in having good spreadability. Additionally, the hair styling product should be easily removable by washing the hair with shampoo. It is difficult to further optimize one or more of the desired properties without impairing the others. It is an objective of the invention to develop a clear non irritating product with a non-liquid, gelwax-like consistency with a combination of good shine and good hold properties when applied to scalp hair.

### SUMMARY OF THE INVENTION

The present invention is directed to a hair styling composition in the form of a microemulsion. The microemulsion is clear or transparent and non-fluid at room temperature and comprises
(A) 20 to 60% by weight water;
(B) at least 3% by weight of an oil phase, said oil phase comprising hydrophobic oil that is liquid at 25 °C and optionally comprises dissolved lipohphilic materials;
(C) at least 20% by weight of at least one emulsifier;
(D) at least one polymer selected from anionic hair fixing polymers and nonionic hair fixing polymers or a combination of both.
Preferred is A hair styling composition comprising a combination of said anionic hair fixing polymer and said nonionic hair fixing polymer

The present invention is further directed to methods of hair treatment using the composition. These and other features, aspects, and advantages of the present invention will become evident to those skilled in the art from a reading of the present disclosure.

### DETAILED DESCRIPTION OF THE INVENTION

The hair styling compositions of the present invention include water, an oil phase, emulsifier and hair fixing polymers. Each of these components, as well as preferred or optional components, is described in detail hereinafter. All percentages, parts and ratios are based upon the total weight of the compositions of the present invention, unless otherwise specified. All such weights as they pertain to listed ingredients are based on the active level and, therefore, do not include solvents or by-products that may be included in commercially available materials, unless otherwise specified. All molecular weights as used herein are weight average molecular weights expressed as grams/mole, unless otherwise specified.

Herein, "comprising" means that other steps and other ingredients which do not affect the end result can be added. This term encompasses the terms "consisting of" and "consisting essentially of". The compositions and methods of the present invention can comprise, consist of, and consist essentially of the essential elements and limitations of the invention described herein, as well as any of the additional or optional ingredients, components, steps, or limitations described herein.

The terms "hydrophobic" and "lipophilic" as used herein, mean substances which are substantially water insoluble, but soluble in the oil phase, with the solubility in the oil phase being higher than that in water or in the aqueous phase. The term "hydrophilic" as used herein, means substances which are substantially water soluble and oil insoluble with the solubility in water or the aqueous phase being higher than that in the oil phase. The term "oil phase" as used herein, means a liquid phase which is seperated from water or the aqueous phase. The term "aqueous phase" as used herein, means a liquid phase which comprises water and can additionally comprise hydrophilic co-solvents and water soluble substances.

Microemulsions are macroscopic homogenous (isotrope), transparent mixtures of two mutually insoluble liquids. Typically, there are fluctuating bi-continuous oil-water domaines besides a droplet structure with typical droplet sizes of about 10 to 200 nm. The microemulsions herein are also physically stable. By "physically stable" it is meant herein that the microemulsions do not show phase separation upon prolonged storage, i.e., the droplets comprising the oil phase remain dispersed in the aqueous phase. The term "non-fluid" as used herein, means compositions that do not flow at ambient conditions, for example they do not run off a sheat of glass tilted at an angle of 45° and at a temperature of 25 °C. Non-fluid compositions can be for example solid, semi-solid, paste-like, cream-like or highly viscous gel-like. The term "gelwax" as used herein, means a composition that has the optically clear or transparent appearance typical for a gel and the haptic and softening properties typical for a wax-like product, e.g. is non-fluid at room temperature but softens or becomes fluid under shear or warming. The viscosity of highly viscous compositions is preferred to be at least 5000 mPa s, more preferred at least 10000 or at least 25000 mPa s, measured with a HAAKE VT-550 Rheometer (SV-DIN, 25°C, shear rate 12,9 s⁻¹).

The term "room temperature" as used herein, means 25°C. The terms "clear" and "optically clear" as used herein, mean compositions in which no cloudiness or haze can be detected with the naked eye. The terms"transparent" as used herein, means compositions or materials which are not perfectly clear but show cloudiness or haze and are translucent, i.e. light shines through as opposed to an opaque or milk appearance typically associated with emulsions.

Hair fixing polymers are polymeric compounds which impart hair-holding or style-retention properties to hair, e.g. when applied as 0,01 to 5% by weight aqueous, alcoholic or aqueous-alcoholic solution or dispersion. In particular, hair fixing polymers are those polymers listed in the International Cosmetic Ingredient Dictionary and Handbook, 10^{th} edition 2004 with the function "Hair Fixatives".

All cited references are incorporated herein by reference in their entireties. Citation of any reference is not an admission regarding any determination as to its availability as prior art to the claimed invention.

### Aqueous Phase

The compostions according to the invention comprises an aqueous phase made up of water and optionally water soluble substances. The amount of water is 20 to 60% by weight, preferred 22 to 40% by weight, based on the total composition. Deionized water is preferably used. Water from natural sources containing mineral cations can also be used, depending on the desired characteristic of the product. Additionally, hydrophilic co-solvents can be comprised. The level and species of the co-solvents are selected according to the compatibility with other components, and other desired characteristics of the product. Preferred are monohydric or polyhydric alcohols which are soluble in the aqueous phase and liquid at room temperature. The amount of alcohol is preferably 0 to 20% by weight, more preferably from 1 to 10% by weight of the total composition. Alcohols can be those conventionally used for cosmetic purposes, e.g. monohydric C1 to C6 alcohols such as ethanol and isopropanol. Ethanol is especially preferred. The pH is preferably in the range of from 6 to 9, more preferably from 6,5 to 8. Buffers and other pH adjusting agents can be included to achieve or stabilise the desirable pH.

In one embodiment of the invention, the hair styling composition additionally contains at least one polyhydric alcohol for further improving the hair shine. The polyhydric alcohols have at least two alcoholic hydroxyl groups. They have preferably 2 to 6 carbon atoms and 2 to 6 hydroxyl groups such as glycerol, C2- to C4-alkylenglycols, and sorbitol. Especially preferred are glycerol and C2- to C4-alkylenglycols, such as ethylenglycol and propylenglycol. The amount of polyhydric alcohol is preferably from 0,1 to 15, more preferably from 0,5 to 10% by weight based on the total composition.

### Oil phase

The amount of oil phase is at least 3% by weight, preferred 4 to 30% or 5 to 20% by weight and comprises hydrophobic oil that is liquid at 25 °C and optionally dissolved lipophilic materials. In one embodiment the oil phase does not contain waxes that are solid at room temperature or these waxes are present only in such minor amounts that do not impair optical clarity, e.g. less thant about 5% or less than about 1% by weight based on the total composition. The hydrophobic oils and the lipophilic materials of the oil phase can for example be selected from vegetable oils, animal oils, mineral oils, silicone oils, hydrocarbon oils, hydrogenated polyolefins, fatty alcohols with at least 8 carbon atoms including branched alcohols such as guerbet alcohols, oils from fatty acids and polyols, oils from fatty acids and monohydric C1- to C30-alcohols (preferred C3- to C22-alcohols) and hydrophobic waxes and mixtures of said hydrophobic compounds. Nonlimiting hydrophobic oils are for example cyclic paraffins, parrafin oils, polydecene, mineral oil, isohexadecane, dodecane, isoeicosane, liquid polydimethylsiloxane, cyclotetrasiloxane, cyclopentasiloxane, phenyltrimethicone, isocetylpalmitate, isopropylmyristate, isopropylpalmitate, isopropylstearate, octylisostearate, octylcocoate, octylpalmitate, octyldodecylmyristate, caprylic/capric triglyceride, butyloctanol, hexyloctanol, butyldecanol, hexyldecanol, octyldodecanol, hexyldecanol, stearylheptanoate, isohexyldecanoate, isodecyloctanoate, dibutyladipate, dicaprylylether, C12-15-alkylbenzoate, hydrogenated polyisobutene, squalane, squalene, native oils such as jojoba oil, olive oil, sunflower oil soja oil, peanut oil, rape seed oil, sweet almond oil, palm-oil, coconut oil, castor oil, hydrogenated castor oil, wheat germ oil, grape seed oil, safflower oil, evening primrose oil, macedemia nut oil, corn oil, avocado oil and similar oils.

Especially preferred oil compounds are mineral oil and branched C8 to C30 alkyl alcohols.

### Emulsifier

Another essential element of the present invention is an emulsifier or a mixture thereof. An emulsifier is needed in order to form the microemulsions according to the present invention because it allows to disperse the oily phase in the aqueous phase of the oil-in-water microemulsions of the present invention. The presence of an emulsifier or mixtures thereof allows to control the size of the droplets comprising said oil phase according to the present invention. It is understood herein that the emulsifier to be used herein or mixtures thereof as well as the levels thereof are chosen, depending on the nature and level of the oil substances, so as to form the microemulsions according to the present invention. The amount of emulsifier is at least 20% by weight, for example 20 to 60% by weight, preferred at least 30% by weight, for example 30 to 50% by weight, based on the total composition.

Suitable emulsifiers to be used in the present invention include any emulsifier known to those skilled in the art as being able to form a microemulsion as defined herein. The emulsifiers include nonionic, anionic, cationic, amphoteric and/or zwitterionic surfactants. Most preferred are nonionic emulsifiers or mixtures of nonionic emulsifiers.

Nonionic emulsifiers are for example
- alkoxylated fatty alcohols such as C8- to C30- or preferably C8- to C22-alcohols, alkoxylated fatty acids or alkoxylated fatty acid glycerides such as C12 to C22-fatty acids, alkoxylated alkylphenols (e.g. alkyl groups with 8 to 15 carbon atoms); typical degrees of ethoxylation being from 2 to 100 or 4 to 30 and typical degrees of propoxylation being from 1 to 5;
- C8 to C30-, preferably C12- to C22-fatty acid glycerolmono- or diester, ethoxylated with from 1 to 30 mole ethylenoxide;
- Castor oil or hydrogenated castor oil ethoxylated with from 5 to 60 mole ethylenoxide;
- Fatty acid sugar ester, especially ester of sucrose with one or two C8- to C30 or C12 to C22-fatty acid, INCI: Sucrose Cocoate, Sucrose Dilaurate, Sucrose Distearate, Sucrose Laurate, Sucrose Myristate, Sucrose Oleate, Sucrose Palmitate, Sucrose Ricinoleate, Sucrose Stearate;
- ester of sorbitan with one, two or three C8- to C22-fatty acid and a degree of ethoxylation of from 4 to 20;
- polyglyceryl fatty acid ester, especially of one, two or more C8- to C22-fatty acids with polyglycerol of preferably 2 to 20 glycerol units;
- alkylglucoside, alkyloligoglucoside or alkylpolyglucoside with C8- to C22-alkyl groups, e.g. Decyl Glucoside oder Lauryl Glucoside.

Amphoteric surfactants are for example amin oxides with at least one hydrocarbon chain with from 6 to 30 carbon atoms. Typical zwitterionic surfactants include betaine and sulphobetaine surfactants with at least one hydrophobic hydrocarbon chain with from 6 to 30 carbon atoms which might include ester or amido groups. Anionic surfactants are for example alkyl carboxylic acids, alkyl ethersulfates, alkylsulfates, sulfosuccinates, fatty acid isethienates, phosphoric acid alkyl ester, ethoxylated phosphoric acid alkyl ester such as mono- di- or triesters of phosphoric acid with C8- to C22-fatty alcohols ethoxylated with 2 to 30 mol ethylenoxide, acylaminoacids, said alkyl groups having preferably 8 to 30 carbon atoms.

Preferred emulsifiers to be used herein are selected from ethoxylated castor oils, ethoxylated hydrogenated casor oils, fatty acid sugar esters, ethoxylated fatty acid monoglycerides and ethoxylated fatty alcohols. Preferred emuslsifiers and emulsifier mixtures are those with NRU₅₀ values of at least 110 µg/ml or above 200 µg/ml or above 750 µg/ml. (NRU₅₀ values can be determined by standard test methods and the standard performance protocol described in E. Borenfreund, J.A. Puerner, Toxicology Letters 24 (1985), pp. 119 - 124, modified for COLIPA International Validation Study on Alternatives to the Draize Rabbit Eye Irritation Test (Brantom et al, 1997) with the change of using human keratinocyte cell line HaCaT instead of the BALB/c 3T3 mouse fibroblasts and treatment in serum-free culture media.). Preferred emulsifier mixtures comprise at least one first emulsifier selected from said ethoxylated castor oils and ethoxylated hydrogenated castor oils and at least one second emulsifier selected from said fatty acid sugar esters and said ethoxylated fatty acid monoglycerides. Preferred emulsifiers based on castor oil are ethoxylated hydrogenated castor oils with a degree of ethoxylation of from 10 to 100, preferred from 20 to 60 such as those with INCI-names PEG-x Castor Oil or PEG-x Hydrogenated Castor Oil, wherein x is the degree of ethoxylation, e.g. PEG-25 hydrogenated castor oil, PEG-35 hydrogenated castor oil, PEG-40 hydrogenated castor oil, PEG-45 hydrogenated castor oil, PEG-54 hydrogenated castor oil or PEG-60 hydrogenated castor oil. Preferred fatty acid sugar esters are for example those with INCI-names Sucrose Cocoate, Sucrose Dilaurate, Sucrose Distearate, Sucrose Laurate, Sucrose Myristate, Sucrose Oleate, Sucrose Palmitate, Sucrose Ricinoleate, Sucrose Stearat. Preferred ethoxylated fatty acid monoglycerides are for example those of formula

CₙH₍₂ₙ₊₁₎-CO-OCHCH(-OH)CH₂(-OCH₂CH₂-)ₓOH

wherein n is a number of from preferably 7 to 29, especially from 11 to 21; x is a number denoting the degree of ethoxylation, e.g. from 2 to 100, or 3 to 40 or 5 to 12; such as those with INCI-names PEG-x Glyceryl Cocoate, PEG-x Glyceryl Isostearate, PEG-x Glyceryl Laurate, PEG-x Glyceryl Oleate, PEG-x Glyceryl Ricinoleate, PEG-x Glyceryl Sesquioleate, PEG-x Glyceryl Stearate, PEG-x Glyceryl Tallowate, wherein x is the degree of ethoxylation.

### Anionic hair fixing polymers

The amount of anionic hair fixing polymer is preferably from 0,1 to 15, more preferably from 0,5 bis 10% by weight based on the total composition. Preferred are polymers which are soluble in water or in the aqueous phase. Anionic polymers are polymers from at least one monomer with at least one pending acidic group that can be neutralized. The monomers with acid groups can be copolymerised with monomers without acid groups. Preferred acid groups are -COOH, -SO₃H, -OSO₃H, -OPO₂H und-OPO₃H₂, carboxylic acid being most preferred. The acid groups can be unneutralised, partially neutralised or completely neutralised. Preferred is a degree of the anionic, neutralised form of from 50 to 100%. Nonlimiting examples of neutralising agents include primary or secondary organic amines, or inorganic bases such as ammonia, NaOH, KOH, ammonium hydroxide etc.. Preferred are amino alcohols with 1 to 10 carbon atoms and 1 to 3 hydroxy groups such as aminomethyl propanol (AMP), monethanolamine, diethanol amine, triethanolamine, tetrahydroxypropyl ethylendiamine, diisopropanolamine, tromethamine, and mixtures thereof. Suitable monomers are ethylenically unsaturated, radically polymerisable compounds carrying at least one acid group, e.g. styrene sulfonic acid, 2-acrylamide-2-methylpropane sulfonic acid or carboxyvinyl monomers like acrylic acid, methacrylic acid, crotonic acid, fumaric acid, maleic acid, maleic anhydride and its monoesters or itaconic acid. Comonomers without acid groups are e.g. acrylamide, methacrylamide, alkyl- and dialkyl acrylamide, alkyl- and dialkyl methacrylamide, alkylacrylate, alkylmethacrylate, vinyl caprolactone, vinyl pyrrolidone, vinyl ester, vinyl alcohol, propylen glycol or ethylen glycol, amine substituted vinyl monomers such as dialkylaminoalkyl acrylate, dialkylaminoalkyl methacrylate, monoalkylaminoalkyl acrylate and monoalkylaminoalkyl methacrylate,
wherein preferred alkyl groups are C1- to C7-alkyl groups, especially C1- to C3-alkyl groups.

Anionic hair fixing polymers are in particular copolymers of acrylic or methacrylic acid with monomers selected from acrylic acid esters, methacrylic acid esters, acrylamides, methacrylamides and vinyl pyrrolidone; homopolymers of crotonic acid; copolymers of crotonic acid with monomers selected from vinyl esters, acrylic acid esters, methacrylic acid esters, acrylamides, methacrylamides. A natural anionic hair fixing polymer is shellac. Anionic hair fixing polymers are for example vinylacetate/crotonic acid copolymer; partially esterified copolymers of vinyl methylether and maleic anhydride; terpolymers of acrylic acid, alkyl acrylate and N-alkyl acrylamide, e.g. acrylic acid/ethyl acrylate/N-t-butyl acrylamide terpolymer; terpolymers of vinyl acetate, crotonic acid and vinyl alkanoate, e.g. vinyl acetate/crotonic acid/vinyl neodecanoate copolymer; copolymers of alkyl acrylamide (especially octyl acrylamide), alkylamino alkyl methacrylate (especially t-butylaminoethyl methacrylate) and two or more monomers selected from acrylic acid, methacrylic acid and their esters, wherein the alkylgroups have from 1 to 4 C-atoms and at least one of the monomers has an acid group; copolymers of acrylic acid, methyl acrylate and methacrylamidopropyl trimethyl ammonium chloride (INCI-name: polyquaternium-47); copolymer of acrylamidopropyl trimethylammonium chloride and acrylates; or copolymers of acrylamide, acrylamidopropyl trimethylammonium chloride, 2-amidopropyl acrylamide sulfonate and dimethylamino propyl amine (INCI-name: polyquaternium-43). Suitable are also polymers with betaine groups, e.g. copolymers of methacryloyl ethylbetaine and two or more monomers selected from acrylic acid and its alkyl esters (INCI-name Methacryloyl Ethyl Betaine/Acrylates Copolymer). Most preferred anionic hair fixing polymer is vinylacetate/crotonic acid copolymer (INCI-name: Vinylacetate/Crotonates Copolymer, marketed for example as Luviset® CA66 by BASF).

### Nonionic hair fixing polymers

The amount of nonionic hair fixing polymer is preferably from 0,1 to 15, more preferably from 0,5 bis 10% by weight based on the total composition. Preferred are polymers which are soluble in water or in the aqueous phase.

Nonionic polymers are polymers without acidic, anionic, basic or cationic groups. Synthetic, non-ionic hair fixing polymers are for example:
Homo- oder copolymers of at least one monomer selected from vinyl pyrrolidone; vinyl caprolactam; vinyl ester e.g. vinyl acetate, vinyl alcohol, acrylamide, methacrylamide, alkyl- and dialkyl acrylamide, alkyl- und dialkyl methacrylamide, dialkylaminoalkyl methacrylamide, dialkylaminoalkyl acrylamide, alkylacrylate, alkylmethacrylate, propylenglycol oder ethylenglykol, wherein preferred alkyl groups of these monomers are C1 - to C7-alkyl groups, more preferred C1 - to C3-alkyl groups. Suitable are e.g. homopolymers of vinyl caprolactam, homopolymers of vinyl pyrrolidone, homopolymers of N-vinyl formamide. Suitable hair fixing polymers are also copolymers of vinyl pyrrolidone and vinyl acetate; terpolymers of vinyl pyrrolidone, vinyl acetate and vinyl propionate; terpolymers of vinyl pyrrolidone, vinyl caprolactam and dialkylamino alkyl (meth)acrylate; terpolymers of vinyl pyrrolidone, vinyl caprolactam and dialkylamino alkyl (meth)acrylamide; polyacrylamide; polyvinyl alcohol; and hair fixing polyethylene glycol/polypropylene glycol copolymers. Preferred are nonionic vinyl lactam homo- or copolymers. Suitable vinyl lactams are e.g. vinyl caprolactam and vinylpyrrolidone. Nonionic hair fixing polymers are for example thos with INCI-names PVP; VP/VA copolymer; VP/Methacrylamide/Vinyl Imidazole Copolymer.

Especially preferred nonionic hair fixing polymers are polyvinyl pyrrolidones which are marketed e.g as Luviskol® K 17, K 30, K 60, K 80, K 85, K90 or K 115 by BASF. According to the invention low molecular weight polyvinyl pyrrolidones with K numbers below 80 are preferred over high molecular weight PVP due to a higher degree of clarity of the end product. More preferred are polyvinyl pyrrolidones with K numbers of 60 or below such as PVP K60 or PVP K 30,most preferred are polyvinyl pyrrolidones with K numbers below 55 such as PVP K30. The preferred PVP with K number below 80 is characterized by a viscosity of less than 60 mPa s of a 5% by weight solution in water at 23 °C measured in a Brookfield RVT viscometer with No. 3 spindle at 100 rpm. The more preferred PVP with K number of 60 or below are characterized by a viscosity of less than 40 mPa s and the most preferred PVP with K number below 55 is characterized by a viscosity of less than 30 mPa s of a 5% by weight solution in water at 23 °C measured in a Brookfield RVT viscometer with No. 3 spindle at 100 rpm.

### Optional ingredients

The composition according to the invention can also contain conventional cosmetic additives usually used in hair treatment compositions, e.g. fragrances and perfume oils in an amount of 0.01 to 1% by weight; preservatives, such as parabenes or iodopropynyl butylcarbamate in an amount of 0.01 1 to 1% by weight; buffer substances, such as sodium citrate or sodium phosphate, in an amount of 0.1 to I % by weight; hair care substances, such as e.g. betaine, panthenol, plant extracts, vegetable extracts, protein hydroylsates and silk hydrolysates, lanolin derivatives, in an amount of 0.1 to 5% by weight; physiologically compatible silicone derivatives, such as volatile or non-volatile silicone oils or high molecular weight siloxane polymers in an amount of 0.05 to 20% by weight; light protective agents, antioxidants, radical-trapping agents, anti-dandruff agents; vitamines; luster-imparting substances and combability-improving substances in an amount of 0.01 to 2% by weight; product coloring agents in an amount of 0.1 to 1% by weight; pigments in an amount of 0.01 to 25% by weight.

The composition according to the invention is preferably clear, transparent or translucent but it can also contain visible pigments or stably dispersed particulate materials. The composition can be colorless or colored by known cosmetic product coloring dyes.

### METHOD OF MAKING

The compositions of the present invention can be made by conventional formulation and mixing techniques. Emulsification of aqueous phase and oil phase is preferably done at elevated temperatures of for example 80 to 100 °C. Volatile ingredients such as fragrances are added preferably at lower temperatures for example at 50 to 70°C. The emulsified composition is filled into the final packaging when still in a fluid state at temperatures above room temperature, for example at 50 to 70°C. The compositions become non-fluid after cooling to room temperature. The final packaging is preferably a transparent or translucent package.

### METHOD OF USE

The method of hair treatment according to the invention comprises the steps of:
a) providing a composition according to the invention;
b) applying said composition to hair; and
c) setting or putting the hair in a hair style.
Such method generally involves application of an effective amount of the product to dry, slightly damp, or wet hair preferably before the hair is arranged to a desired style. The composition is then dried or allowed to dry. By "effective amount" is meant an amount sufficient to provide the hair hold and style benefits desired considering the length and texture of the hair. In general, from about 0.5g to about 50g of product will be applied to the hair, depending upon the particular product formulation, length of hair, and type of hair style.

The compositions of the present invention are used to provide the hair styling/holding and product benefits of the present invention, in particular for increasing at least one of
- Strong hold
- Outstanding shine
- Structure and definition
- Antifrizz effect
- Clear appearance
- Pleasant feel of product mass
- Easy to distribute in hand
- Easy to work into hair
- Well suited for short structured styles (for men as well as women)
- Relative low overloading of the hair compared with conventional gelwax
- Easy to wash out.

### EXAMPLES

The compositions illustrated in the following examples illustrate specific embodiments of the hair styling compositions of the present invention, but are not intended to be limiting thereof. Other modifications can be undertaken by the skilled artisan without departing from the spirit and scope of this invention. These exemplified embodiments of the hair styling composition of the present invention provide styling and shine benefits. The compositions illustrated in the following examples are prepared by conventional formulation and mixing methods. All exemplified amounts are listed as weight percents and exclude minor materials such as diluents, preservatives, color solutions, imagery ingredients, botanicals, and so forth, unless otherwise specified. If a trade name is mentioned as ingredient and the respective product is itself a mixture (e.g. a solution, emulsion, dispersion etc.), then the exemplified amount relates to this mixture, unless otherwise specified.

### Example 1 Non-fluid, transparent hair styling microemulsion

| | |
|---|---|
| 0,5 | Vinylacetate/Crotonates Copolymer |
| 0,7 | Polyvinylpyrrolidone K 30 (Luviskol® K 30) |
| 13,5 | Mineral Oil |
| 0,5 | 2-Octyl-dodecanol |
| 5,0 | Propylene Glycol |
| 23,4 | PEG-40 Hydrogenated Castor Oil |
| 14,0 | PEG-25 Hydrogenated Castor Oil |
| 3,9 | PEG-200 Hydrogenated Glyceryl Palmate |
| 2,5 | Ceteareth-20 |
| 1,0 | PEG-7 Glyceryl Cocoate |
| 0,3 | Panthenol |
| 0,11 | Aminomethylpropanol (95%) |
| q.s. | Fragrance, preservatives, color |
| Ad 100 | Water |

### Example 2 Non-fluid, transparent hair styling microemulsion

| | |
|---|---|
| 0,25 | Vinylacetate/Crotonates Copolymer |
| 1,25 | VP/METHACRYLAMIDE/VINYL IMIDAZOLE COPOLYMER |
| 12,0 | Mineral Oil |
| 1,0 | 2-Octyl-dodecanol |
| 5,0 | Propylene Glycol |
| 24,0 | PEG-40 Hydrogenated Castor Oil |
| 14,0 | PEG-25 Hydrogenated Castor Oil |
| 3,9 | PEG-200 Hydrogenated Glyceryl Palmate |
| 2,0 | Steareth-20 |
| 1,0 | PEG-7 Glyceryl Cocoate |
| 0,03 | Aminomethylpropanol (95%) |
| q.s. | Fragrance, preservatives, color |
| Ad 100 | Water |

### Example 3 Non-fluid, transparent hair styling microemulsion

| | |
|---|---|
| 0,25 | Vinylacetate/Crotonates Copolymer |
| 0,5 | Polyvinylpyrrolidone K 60 |
| 11,0 | Mineral Oil |
| 1,5 | 2-Octyl-dodecanol |
| 3,0 | Propylene Glycol |
| 22,0 | PEG-40 Hydrogenated Castor Oil |
| 13,0 | PEG-25 Hydrogenated Castor Oil |
| 3,9 | PEG-200 Hydrogenated Glyceryl Palmate |
| 2,3 | Ceteareth-20 |
| 1,0 | PEG-7 Glyceryl Cocoate |
| 0,03 | Aminomethylpropanol (95%) |
| q.s. | Fragrance, preservatives, color |
| Ad 100 | Water |

The exemplary microemulsions have benefits over conventional hair styling microemulsion or conventional gelwaxes in one or more of strong hold of treated hair, outstanding shine of treated hair, structure and definition of treated hair, antifrizz effect of treated hair, clear product appearance, pleasant feel of product mass, easy to distribute in hand, easy to work into hair, well suited for short structured hir styles (for men as well as women), relative low overloading of treated hair, easy to wash out.

## Claims

1. A hair styling composition in the form of a microemulsion, said microemulsion being clear or transparent and non-fluid at room temperature and comprising
(A) 20 to 60% by weight water;
(B) at least 3% by weight of an oil phase, said oil phase comprising hydrophobic oil that is liquid at 25 °C and optionally comprises dissolved lipophilic materials;
(C) at least 20% by weight of at least one emulsifier;
(D) at least one polymer selected from anionic hair fixing polymers and nonionic hair fixing polymers or a combination of both.

2. A hair styling composition of of any preceding claim comprising a combination of at least one of said anionic hair fixing polymer and at least one of said nonionic hair fixing polymer.

3. The composition of claim 1 comprising from 22 to 40% by weight of water; 5 to 20% by weight of said oil phase; at least 30% by weight of said at least one emulsifier; 0,1 to 15 % by weight of said first polymer; and from 0,1 to 15 % by weight of said second polymer.

4. The composition of any preceding claim further comprising at least one monohydric or polyhydric alcohol which is soluble in the aqueous phase and liquid at room temperature.

5. The composition of the preceding claim, wherein the polyhydric alcohol is selected from glycerol and C2- to C4-alkylen glycols.

6. The composition of any preceding claim, wherein the hydrophobic oils and the lipophilic materials of the oil phase are selected from vegetable oils, animal oils, mineral oils, silicone oils, hydrocarbon oils, hydrogenated polyolefins, branched alcohols with at least 8 carbon atoms, fatty alcohols with at least 8 carbon atoms, oils from fatty acids and polyols, oils from fatty acids and monohydric C1- to C30-alcohols and hydrophobic waxes.

7. The composition of any preceding claim, wherein the oil phase does not contain wax solids in an amount that impairs optical clarity.

8. The composition of any preceding claim, wherein the emulsifier is selected from ethoxylated castor oils, ethoxylated hydrogenated castor oils, fatty acid sugar esters, ethoxylated fatty acid monoglycerides and ethoxylated fatty alcohols.

9. The composition of the preceding claim, wherein the emulsifier is selected from castor oils with a degree of ethoxylation of from 10 to 100; hydrogenated castor oils with a degree of ethoxylation of from 10 to 100; esters of sucrose and C8- to C30-fatty acids; monoglycerides of C8- to C30-fatty acids and with a degree of ethoxylation of from 2 to 100; C8- to C30 fatty alcohols with a degree of ethoxylation of from 2 to 100.

10. The composition of the preceding claim, wherein the emulsifier is selected from PEG-25 hydrogenated castor oil, PEG-35 hydrogenated castor oil, PEG-40 hydrogenated castor oil, PEG-45 hydrogenated castor oil, PEG-54 hydrogenated castor oil, PEG-60 hydrogenated castor oil, sucrose cocoate, sucrose dilaurate, sucrose distearate, sucrose laurate, sucrose myristate, sucrose oleate, sucrose palmitate, sucrose ricinoleate, sucrose stearate, monoglycerides of C 12- to C22-fatty acids and with a degree of ethoxylation of from 3 to 40.

11. The composition of any preceding claim wherein the anionic hair fixing polymer is selected from copolymers of vinylacetate and crotonic acid; copolymers of octylacrylamide, acrylic acid, butylamino methacrylate, methyl methacrylate and hydroxypropylmethacrylate; copolymers of alkylacrylate, acrylic acid and alkylacrylamide.

12. The composition of any preceding claim wherein the nonionic hair fixing polymer is selected from polyvinylpyrrolidone; copolymers of vinylpyrrolidone and vinylacetate; copolymers of vinylpyrrolidone, methacrylamide and vinylimidazol.

13. The composition of any preceding claim wherein the anionic hair fixing polymer is selected from copolymers of vinylacetate and crotonic acid and wherein the nonionic hair fixing polymer is selected from polyvinylpyrrolidone.

14. The composition of any preceding claim, wherein the nonionic hair fixing polymer is selected from low molecular weight polyvinylpyrrolidones, **characterized by** a Brookfield viscosity of less than 60 mPa s of a 5% by weight solution in water at 23 °C measured in a Brookfield RVT viscometer with No. 3 spindle at 100 rpm.

15. The composition of any preceding claim, further comprising a transparent or translucent package and wherein said composition is contained in said package.

16. A method of hair treatment, said method comprising the steps of:
a) providing a composition according to any of the composition claims;
b) applying said composition to hair; and
c) setting or putting the hair in a hair style.
